Europäisches Patentamt

European Patent Office

Office européen des brevets

(19)

(11) Veröffentlichungsnummer : **0 342 524 B1**

(12)

# EUROPÄISCHE PATENTSCHRIFT

(45) Veröffentlichungstag der Patentschrift :
**12.08.92 Patentblatt 92/33**

(51) Int. Cl.$^5$ : **A61K 33/42,** // (A61K33/42, 33:14, 31:725, 31:70, 31:19, 31:045)

(21) Anmeldenummer : **89108506.0**

(22) Anmeldetag : **12.05.89**

(54) **Mittel zur Behandlung mykotischer, mikrobischer und anderer gewebsschädigender und pathologischer Manifestationen und Gewebsdeformationen.**

Die Akte enthält technische Angaben, die nach dem Eingang der Anmeldung eingereicht wurden und die nicht in dieserPatentschrift enthalten sind.

(30) Priorität : **13.05.88 DE 3816442**

(43) Veröffentlichungstag der Anmeldung :
**23.11.89 Patentblatt 89/47**

(45) Bekanntmachung des Hinweises auf die Patenterteilung :
**12.08.92 Patentblatt 92/33**

(84) Benannte Vertragsstaaten :
**AT BE CH ES FR GB GR IT LI NL SE**

(56) Entgegenhaltungen :
**EP-A- 0 008 052**
**DE-A- 2 351 512**

(73) Patentinhaber : **Tomic, Dobrivoje, Dr.**
**Fasangartenstrasse 159**
**W-8000 München 90 (DE)**

(72) Erfinder : **Tomic, Dobrivoje, Dr.**
**Fasangartenstrasse 159**
**W-8000 München 90 (DE)**

## Beschreibung

Die Erfindung betrifft Mittel, die zur Behandlung und Vorbeugung mykotischer, mikrobischer, pathologischer und sonstiger gewebsschädigender Manifestationen und Einwirkungen, Gewebsveränderungen und Gewebsdeformationen, zellulärer Stoffwechselstörungen, Ätzungen, Juckreiz und sonstiger Verletzungen der menschlichen und tierischen Gewebe, auch in Genital- und Mundbereich und Unterhautgewebe Anwendung findet und die die in den Ansprüchen angegebene Zusammensetzungen und Anwendungen aufweisen.

Die Grundlage für die bekannten Antimykotika und Antimikrobika bilden in der Regel Antibiotika, Kortison, Kortikoide, Hydroxybenzoate, Ekonazolnitrat, Mykonazolnitrat, Dexamethaison, Ethanole und Prednisolon. Auch die sonstigen Dermatika weisen die gleiche oder ähnliche Zusammensetzung auf. Die bekannten Mittel sind jedoch mit zahlreichen Nachteilen verbunden, da sie, wenn überhaupt, nur bei sehr langer Anwendung eine therapeutische Wirkung aufzeigen. Dabei kommt es oft zu Austrocknung der behandelten Stellen, Hautrötung, Bläschenbildung, Brennen, Juckreiz, Hautatrophie und Allergie. Auch Pseudoheilung und rezidive Prozesse sind nicht selten. In vielen Fällen sind diese bekannten Mittel wegen ihrer bedenklichen und schädlichen Zusammensetzung sogar kontraindiziert oder überhaupt nicht anwendbar wie das z.B. während der Schwangerschaft, bei Säuglingen oder beim Übergangsepithel (z.B. Lippen, Genitalbereich, Augen oder Mundhöhle) der Fall ist. Auch die Haftbarkeit dieser Mittel ist oft nicht oder nur gering vorhanden, was für die Wirkung des Mittels und wegen einer eventuellen Verschmutzung von Kleidung von grossem Nachteil ist.

Aufgabe der Erfindung ist es, Mittel zur Behandlung und Vorbeugung mykotischer, mikrobischer, pathologischer und sonstiger gewebsschädigender Manifestationen und Einwirkungen, Gewebsveränderungen und Gewebsdeformationen, Zellulärer Stoffwechselstörungen, Ätzungen, Juckreiz und sonstiger Verletzungen der menschlichen und tierischen Gewebe, auch in Genital- und Mundbereich und Unterhautgewebe bereitzustellen, die sich durch rasche Wirkung, ausgezeichnete Verträglichkeit, sichere Haftung und leichtes Abwaschen, einen breiten Anwendungsbereich und grosse Tiefenwirkung auszeichnen. Welche sich völlig unbedenklich zur Behandlung, Vorbeugung und Heilung von geschlossenen und offenen Haut-, Schleimhaut- und Gewebsflächen und Stellen eignen. Diese Aufgabe wird durch die Erfindung gelöst. Der Stand der Technik kennt Mittel mit entzündungskemmender und blutstillender Wirhung (EP-A-8 052).

Der Erfindung liegen spezielle Zusammensetzungen zugrunde. Je nach Anwendungszweck und Körperbereich weist jede erfindungsgemäße Mischung einen vergleichweise niedrigeren bis physiologisch normalen pH-Wert und einen vergleichsweise höheren Osmotischen Druck auf.Die erfindungsgemäßen Mittel bestehen aus Alkali- und Erdalkalimetallen und Mineralsalzen als wirkungsvolle Basiskomponenten, aus Binde- und Haftmittel, adstringierenden Mitteln und/oder Säure,Öl (Äth.Öle), Feuchtigkeitsmitteln, denen gegebenenfalls auch ein oder mehrere übliche antimykotische, antimikrobische und sonstige zweckentsprechende Stoffe und Hilfskomponenten beigefügt werden können. Die Mittel sind gekennzeichnet durch die Anwesenheit von Magnesiumperoxid ($MgO_2$). Als Alkali- und Erdalkalimetealle und Mineralsalze werden Verbindungen und Salze aus Na, K, Cl, Mg, Ca und Phosphat bevorzugt, die als Elektrolyten viele Aufgaben erfüllen. Als Binde- und Haftmittel eignen sich unter anderem besonders gut Pektin, als Adstringent vorzugsweise Tannin, als Feuchtigkeitsmittel vorzugsweise Glycerin und als Geschmack- und Kühlmittel Pfefferminzöl. Der synergistische Effekt aller Komponenten bewirkt eine überraschende therapeutische Wirkung der erfindungsgemäßen Mittel. Zur Einstellung des pH-Wertes kann zusätzlich eine Säure wie Phosphor- oder Zitronensäure beigemischt werden. Der Mengenanteil unter den Komponenten ist unterschiedlich und kann je nach Anwendungszweck und Art für eine Komponente 0,1% und für eine andere 18% und mehr betragen. Die angegebenen Beispiele erläutern dies näher. In den angegebenen Mischungen der erfindungsgemäßen Mittel können auch gegebenenfalls Antibiotika, Bakteriostatika, Kortikosteroide, Kortison, Ekonazolnitrat, Dexamethason, Hydroxybenzoate, Vitamine, Farbstoffe, Fett, Wachs, Emulgatoren, Silikone, Polyethylene, Methakrylverbindungen, Talkum, Stärke, Zinkoxyd, Wismut, Vaseline, Dextrose, Saccharide, Paraffine, Säure, Konservierungsstoffe und/oder andere zweckgebundene Mittel und Hilfskomponenten enthalten sein. Die erfindungsgemäßen Mittel können in ihrer Konsistenz einer Paste, Kreme, Salbe, Emulsion, Lösung, Gel, Puder, Spray, Gelatine oder Schaum ähnlich sein. Die Herstelung der erfindungsgemäßen Mittel erfolgt durch Einverleiben und Vermischen der einzelnen Komponenten auf bekannte, auf pharmazeutischem Gebiet übliche Weise.

Die erfindungsgemäßen Mittel unterscheiden sich in ihren Zusammensetzungen, Anwendungsmöglichkeiten, Tolerabilität und therapeutischen Wirkungsefekten grundlegend von allen bekannten vergleichbaren Mitteln.

Es ist als völlig überraschend anzusehen, daß mit den angegebenen erfindungsgemäßen Mitteln, z.B. aus einer Mischung aus $MgO_2$ Alkali- und Erdalkalimetallen und Mineralsalzen, Pektin, Tannin, Pfefferminzöl und Glycerin bestehend, eine schnelle, optimale und sichere therapeutische Wirkung erzielt wird. Die erbrachten Labor- und klinische Ergebnisse bestätigen dies.

Die erfindungsgemäßen Mittel haften sicher und wirkungsvoll auf feuchten und trockenen Flächen und bilden nach ca. 15 min. einen zuverlässigen Schutz über die behandelten Stellen,- eine Art Verband-, was bei bettlägerigen Patienten oder beim Tragen von Kleidung von grossem Vorteil ist. Die neuen erfindungsgemäßen Mittel sind eine Nährmischung für Gewebszellen, insbesondere deshalb, weil die anwesenden Mineralsalze für den Zellstoffwechsel und gute Durchblutung sorgen. Die bekannten Antimykotika und Antimikrobika hingegen bewirken in erster Linie eine Desinfektion auf Kosten lebender Gewebszellen. Die erfindungsgemäßen Mittel eignen sich als besonders wirksam auch bei Gewebsschädigenden Manifestationen, zellulären Stoffwechselstörungen und sonstigen Veränderungen mit Akkumulationen von Gewebsflüßigkeit, Fett und anderen Zellprodukten und- "abfällen", die sich auch als Verdickung, Oedem, Zellulit, Hornhaut, Hämatome oder änlichem manifestieren können.

Die folgenden Beispiele sollen die Erfindung und Ansprüche häher erläutern. Diese Beispiele zeigen einige bevorzugten Zusammensetzungen der erfindungsgemäßen Mittel.

| Komponente | Gehalt in gr. | ungefährer Gehalt in % |
|---|---|---|
| $Na_2HPO_4 \cdot 12\,H_2O$ | 6,5 | 0,7 |
| NaCl | 7,0 | 0,7 |
| $MgCl \cdot 6\,H_2O$ | 0,2 | 0,02 |
| KCl | 0,2 | 0,02 |
| $MgO_2$ | 2,8 | 0,28 |
| Glycerin | 80,0 | 8,0 |
| Tannin | 15,0 | 1,5 |
| Pektin | 100,0 | 10,0 |
| $Ca_3(PO_4)_2$ | 130,0 | 13,0 |
| Pfefferminzöl | 2.5 | 0,2 |
| $H_2O$ | | ad 100 |
| Tego-Betain® L 7 | 40,0 | 4,0 |

| Komponente | Gehalt in gr | ungefährer Gehalt in % |
|---|---|---|
| $Na_2HPO_4 \cdot 12\,H_2O$ | 6,2 | 0,6 |
| NaCl | 6,7 | 0,7 |
| KCl | 0,17 | 0,02 |
| $MgCl \cdot 6\,H_2O$ | 0,17 | 0,02 |
| $MgO_2$ | 2,0 | 0,2 |
| Tannin | 10,0 | 1,0 |
| Glycerin | 120,0 | 12,0 |
| Pektin | 60,0 | 6,0 |
| $Ca_3(PO)_4$ | 60,0 | 6,0 |
| Pfefferminzöl | 2,0 | 0,2 |
| $H_2O$ | | ad 100 |

| | | |
|---|---|---|
| $Na_2HPO_4 \cdot 12H_2O$ | 18,0 | 1,8 |
| NaCl | 20,0 | 2,0 |
| KCl | 1,0 | 0,1 |
| $MgCl_2 \cdot 6H_2O$ | 1,0 | 0,1 |
| $MgO_2$ | 3,0 | 0,3 |
| Glycerin | 160,0 | 16,0 |
| Tannin | 16,0 | 1,6 |
| Pektin | 180,0 | 18,0 |
| $Ca_3(PO)_4$ | 150,0 | 15,0 |
| Pfefferminzöl | 3,0 | 0,3 |
| $H_2O$ | | ad 100 |

Zu den Beispielen 1 bis 3 können, je nach Anwendungszweck, ein anderes Adstringens, und/oder Öl, und/oder Säure, und/oder Emulgatoren, sowie Vitamine, Antibiotika, Bakteriostatika, Konservierungsmittel, Ampholytseifen, Sorbitverbindungen und/oder sonstige im Text beschriebene Stoffe und Mittel dazu oder an Stelle einer oder mehreren Komponenten beigemischt werden.

**Patentansprüche**

1. Mittel mit folgenden Komponenten:
a) einem oder mehreren Alkali- und Erdalkalimetallen und Mineralsalzen
b) einem adstringierenden Mittel, einem Binde- und Haftmittel, einem Feuchtigkeitsmittel und einem Ätherischen Oel
c) gegebenenfalls einem oder mehreren üblichen antimykotischen, antimikrobischen und sonstigen zweckentsprechenden Komponenten
gekennzeichnet durch einen Gehalt an Magensium peroxid ($MgO_2$).

2. Mittel nach Anspruch 1, dadurch gekennzeichnet, daß es 0,2 - 0,4% $MgO_2$ enthalt.

3. Mittel nach Anspruch 1 und 2, dadurch gekennzeichnet, daß die Komponente a) Na-, K-, Cl-, Mg-, Ca- und Phosphatverbindungen oder mindestens eine dieser Verbindungen enthält.

4. Mittel nach Anspruch 1, 2 und 3, dadurch gekennzeichnet, daß die Komponente b) als Adstringens Tannin oder Säure ist, als Binde- und Haftmittel Pektin oder Fett ist, als Äth. Oel Pfefferminzöl oder Zitronenöl oder mindestens eine dieser Komponenten enthält.

5. Mittel nach Anspruch 1 bis 4, dadurch gekennzeichnet, daß die Komponente c) gegebenenfalls Antibiotika, Bakteriostatika, Kortikosteroide, Kortison, Ekonazolnitrat, Dexamethason, Hydroxybenzoat, Vitamine, Farbstoff, Wachs, Emulgatoren, Stärke, Zinkoxyd, Wismut, Vaseline, Dextrose, Saccharide, Paraffine,Säure,Ampholytseifen,Sorbitverbindungen oder Konservierungsmittel enthält.

6. Mittel nach Anspruch 1 bis 4, dadurch gekennzeichnet, daß mindestens 0,6 - 2% $Na_2HPO_4 \cdot 12H_2O$; 0,6 - 2% NaCl; 0,02 - 0,2% KCl; 0,02 - 0,2% $MgCl \cdot 6H_2O$; 0,2 - 0,4% $MgO_2$; 0,5 - 1,6% Tannin; 2,0 - 20% Glycerin; 3,0 - 20% Pektin; 3,0 - 20% $Ca_3(PO)_4$; 0,1 - 0,4% Pfefferminzöl in einer Mischung enthalten sind.

7. Mittel nach Anspruch 1 bis 6, dadurch gekennzeichnet, daß der pH-Wert 2,5 bis 7,8 beträgt und der Osmotische Druck höher als 20 Atm. ist.

8. Mittel nach Anspruch 1 bis 7, dadurch gekennzeichnet, daß sie zur Vorbeugung und Behandlung mykotischer,mikrobischer, pathologischer und sonstiger gewebsschädigender Manifestationen und Einwirkungen, Gewebsveränderungen und Gewebsdeformationen und zellulärer Stoffwechselstörungen, Anwendung finden.

9. Mittel nach Anspruch 1 bis 8, dadurch gekennzeichnet, daß es in Form einer Paste, Creme, Salbe, Gel, Emulsion, Lösung, Puder, Spray, Gelatine oder Schaum sein kann.

**Revendications**

1. Moyen avec les composants suivants:

a) un ou plusieurs composés de métaux alcalins ou alcalino-terreux,

b) un moyen astringent, un liant et adhésif, un humidifiant et une huile éthéré,

c) éventuellement un ou plusieurs des communes composants antimycotiques ou antimicrobiens, caractérisé par le contenu de peroxyde de magnésium ($MgO_2$).

2. Moyen selon la revendication 1, caractérisé en ce qu'il contient 0,2-0,4% de $MgO_2$.

3. Moyen selon les revendications 1 et 2, caractérisé en ce que le composant a) contient des composés de Na, K, Cl, Mg ou des phosphates, ou au moins un de ces composés.

4. Moyen selon les revendications 1, 2 et 3, caractérisé en ce que le composant b) contient comme astringent du tannin ou un acide, comme liant et adhésif de la pectine ou de la graisse, comme huile éthéré huile de menthe poivrée ou de citron, ou au moins un de ces composants.

5. Moyen selon les revendications 1-4, caractérisé en ce que le composant c) contient éventuellement: antibiotiques, bactériostatiques, corticostéroïdes, cortisone, nitrate d'éconazole, dexaméthason, hydroxybenzoates, vitamines, colorants, cire, émulsifiants, amidon, oxyde de zinc, bismuth, vaseline, dextrose, saccharides, paraffine, acides, savons ampholytiques, dérivés du sorbitol ou conservants.

6. Moyen selon les revendications 1-4, caractérisé en ce qu'il contient au moins 0,6-2% de $Na_2HPO_4.12H_2O$; 0,6-2% de NaCl; 0,02-0,2% de KCl; 0,02-0,2% de $MgCl_2.6H_2O$; 0,2-0,4% de $MgO_2$; 0,5-1,6% de tannin; 2,0-20% de glycérine; 3,0-20% de pectine; 3,0-20% de $Ca_3(PO_4)2$; 0,1-0,4% d'huile de menthe poivrée.

7. Moyen selon les revendications 1-6, caractérisé en ce que son pH est 2,5-7,8 et sa pression osmotique est plus élevée que 20 atm.

8. Moyen selon les revendications 1-7, caractérisé en ce que il est employé pour la prévention et le traitement des symptomatologies mycotiques, microbiennes, pathologiques ou nuisibles pour les tissus, des altérations ou déformations des tissus et des troubles du métabolisme cellulaire.

9. Moyen selon les revendications 1-8, caractérisé en ce qu'il peut se présenter en forme de pâte, crème, pommade, gel, émulsion, solution, poudre, spray, gelée ou mousse.

**Claims**

1. Substance with the following components:

a) one or more alkali or earth alkali metal compounds

b) one adstringent substance, one adhesive and binding substance, one moisturizing substance and one etheric oil.

c) if necessary, one or more of the commonly used antimycotic, antimicrobic and other components of similar purpose.

2. Substance according to claim 1, marked in such a way; that it contains 0,2 - 0,4 % of magnésium peroxide ($MgO_2$).

3. Substance according to claims 1 and 2, marked in such a way that component a) be $Na^-$, $K^-$, $Cl^-$, $Mg^-$, Ca and phosphate compounds or at least one of these compounds.

4. Substance according to claims 1, 2, and 3, marked in such a way, that component b) contain tannin or an acid as an adstringent, pectin or a fat as the binding and adhesion substance, mint oil or lemon oil as the etheric oil or at least one of these components.

5. Substance according to claims 1 thru 4, marked in such way, that component c) if necessary contain antibiotics, bacteriostatics, corticosteroids, cortisone, econazolnitrate, dexamethasone, hydroxybenzoate, vitamins, coloring substance, wax, emulgators, starch, zincoxyde, wismut, vaseline, dextrose, saccharide, paraffin, acid ampholitic soaps, sorbit compounds or preservatives.

6. Substance according to claims 1 thru 4, marked in such way, that at least 0,6 - 2 % $MgCl·6H_2O$; 0,2 - 0,4 % $MgO_2$; 0,5 - 1,6 % tannin; 2,0 - 20 % glycerine; 3,0 - 20 % pectin; 3.0 - 20 % $Ca_3(PO)_4$; 0.1 - 0.4 % mint oil be contained in a blend.

7. Substance according to claims 1 thru 6, marked in such way, that the pH value lie in the range from 2.5 to 7.8 and that the osmotic pressure be higher than 20 Atm.

8. Substance according to claims 1 thru 7, marked in a way that it finds application in the prevention and treatment of mycotic, microbiotic, pathological and otherwise tissue-damaging manifestations and influences, tissue alterations and tissue deformations and disturbances and disturbances in cell metabolism.

9. Substance according to claims 1 thru 8, marked in such way, that it can be in the form of a paste, cream salve, gel, emulsion, powder, spray, gelatine or foam.